# EUROPEAN PATENT APPLICATION

(11) **EP 4 292 524 A1**
(43) Date of publication of application: **20.12.2023**
(21) Application number: 22179649.3
(22) Date of filing: 17.06.2022
(51) Int. Cl.: A61B 5/026, A61B 5/0295, A61B 5/1455, A61B 5/00

(54) **INTRACRANIAL CATHETER FOR MEASURING A FLOW OF BLOOD**

(71) Applicant: CARAG AG, 6340 Baar (CH)
(72) Inventor: Schenk, Daniel, 8910 Affoltern am Albis (CH)
(74) Representative: Grünecker Patent- und Rechtsanwälte PartG mbB

(57) **Abstract**

The present invention relates to an intracranial catheter (2) for measuring a flow of blood through a body tissue, comprising a longitudinal body (4) adapted to be inserted into the craniospinal compartment, which longitudinal body (4) has a tip element (6), which forms a distal end of said longitudinal body (4), and preferably a channel extending from a proximal end of said longitudinal body (4) to at least one opening (18) of said tip element (6), and a sensor (22) configured to detect a pulsatile pressure curve, which is analyzed via a pulse contour analysis performed by a processing unit (44) coupled to said sensor (22).

## Description

The present invention relates to an intracranial catheter for measuring a flow of blood through a body tissue, wherein the intracranial catheter comprises a longitudinal body, which is adapted to be inserted into the craniospinal compartment and which has a tip element forming a distal end of said longitudinal body. Such an intracranial catheter is known, for example, from US 5 024 226 A.

US 5 024 226 A relates to an intracranial catheter comprising a sensor for determining the oxygen availability of tissues within the skull, wherein the sensor is placed epidurally through the skull to measure oxygen availability. During neurological and neurologically related surgical procedures, it is oftentimes desirable to continuously monitor the oxygenation of blood which is supplied to the brain. Frequently, access is gained to the brain through a borehole in the skull, and a sensor which optically measures oxygenation can then be inserted through such a borehole. An optical sensor should then exhibit numerous design and performance criteria in order to operate satisfactorily in this environment. The sensor must be capable of insertion through the borehole so as to contact tissue where oxygen availability is to be measured. The sensor must be soft so that it does not damage neurological tissue, yet be sufficiently rigid in certain dimensions so that it can be maneuvered from outside the skull. It also must be sized to fit inside the borehole and in the location where measurements are to be taken. Furthermore, the sensor must be designed so as to eliminate detection of ambient light which will interfere with detection of the desired optical signals. The sensor must also prevent the detection of directly transmitted light from the light source of the sensor.

While US 5 024 226 A focuses on monitoring oxygen availability in a cerebral blood flow by an optical measurement, it is an object of the present invention to measure a cerebral blood flow (CBF).

As a solution to the above object, the present invention proposes an intracranial catheter for measuring a flow of blood with the features of claim 1.

This intracranial catheter is characterized by a sensor configured to detect a pulsatile pressure curve, which is analyzed via a pulse contour analysis performed by a processing unit coupled to said sensor, said sensor preferably comprising a plurality of sensor devices. As the blood volume is proportional to the area under the systolic part of the pressure curve, analyzing the pulsatile pressure curve via pulse contour analysis allows to determine a cerebral blood flow.

The viscoelasticity of blood vessels also affects the cerebral blood flow. The Moens-Koteweg equation models the relationship between the incremental elastic modulus of the arterial wall and the pulse wave velocity (PWV). PWV in m/s can be measured by using the signals of the sensor devices, by taking advantage of the distance between them.

Using an absolute CBF measurement done e.g. by indocyanine green spectroscopy, or CT or MRI perfusion measurement, and the calculated patient specific viscoelasticity, the pulse contour algorithm can be calibrated to calculate absolute CBF values, updated for each heartbeat, i.e. continuous.

Preferably, the sensor comprises a pressure sensor device configured to detect the pulsatile pressure curve. In particular, the pulsatile pressure curve can be detected using an absolute pressure sensor, a gauge pressure sensor, a vacuum pressure sensor, a differential pressure sensor, or a sealed pressure sensor. The working principle of the pressure sensor device is not particularly limited by the present invention. For example, an optical pressure sensor can be used. Alternatively or additionally, the pulsatile pressure curve can be derived from an optical absorption measurement, in particular by using a photoplethysmogram.

The sensor can be provided in or on the longitudinal body, whereas the processing unit can be provided at a certain distance to the longitudinal body. For example, the processing unit can be provided by an external device which is coupled to the sensor for receiving the sensor signals via cable or wirelessly. However, in one embodiment, the processing unit can be provided within the longitudinal body.

Preferably, a channel is formed in the longitudinal body, wherein the channel extends from a proximal end of said longitudinal body to at least one opening of said tip element. Said channel may be used as a drainage channel for cerebrospinal fluid. In one embodiment of the present invention, however, the tip element may not have an opening and/or the longitudinal body may not have a channel. The longitudinal body may be formed in a similar or the same way as the longitudinal body disclosed in US 5 024 226 A or WO 2016/192958 A1, the content of said documents being incorporated herein by reference.

In a preferred embodiment of the intracranial catheter, the sensor comprises optical sensor devices including at least one light emitter for emitting light, preferably in the near infrared range, into the body tissue and at least one light receiver for receiving light reflected in the body tissue and back to the longitudinal body and for generating a signal. It is preferable to use a light source with coherent light in the near infrared range, which is able to penetrate biological tissue. It is very preferable to specifically use wavelengths that are suitable for a chosen measurement technique. Light with wavelengths of 690 nm, 760 nm, 808 nm, and 905 nm are preferably used.

Typically, indocyanine green is used in medical diagnostics as an indicator substance, which is administered intravenously. The absorption and fluorescence spectrum of indocyanine green is in the near infrared region. Both depend largely on the concentration, and the concentration profile measured by the intracranial catheter strongly depends on the blood flow.

The optical sensor devices are preferably arranged in a recess formed in the longitudinal body and covered by a translucent window. The pressure sensor device can be arranged in a similar recess. The recesses may be formed and the optical sensor devices and the pressure sensor device can be arranged in a similar or the same way as the ones disclosed in WO 2016/192958 A1, the content of which is incorporated herein by reference.

In a preferred embodiment of the intracranial catheter, the signal generated by the light receiver comprises information about a difference between an oxyhemoglobin concentration and a deoxyhemoglobin concentration, which signal is processed to monitor continuous cerebral blood flow.

In a further preferred embodiment, the intracranial catheter comprises a first set of at least two LEDs, wherein a first LED of said first set emits light at different wavelength than a second LED of said first set and wherein said LEDs of said first set are adapted to emit light in the near infrared range and wherein a light receiver adapted to receive the reflected light of said first set generates a signal, which signal is processed to monitor cerebral blood flow. Specifically, it is preferable that the first LED of said first set emits light with a wavelength of 808 nm and the second LED of said first set emits light with a wavelength of 905 nm.

In a further preferred embodiment, the intracranial catheter comprises a second set of at least two LEDs, wherein a first LED of said second set emits light at different wavelength than a second LED of said second set and wherein said LEDs of said second set are adapted to emit light in the near infrared range and wherein a light receiver adapted to receive the reflected light of said second set generates a signal, which signal is processed to monitor cerebral oxygen saturation. Specifically, it is preferable that the first LED of said second set emits light with a wavelength of 690 nm and the second LED of said second set emits light with a wavelength of 760 nm or 905 nm.

In a further preferred embodiment, the intracranial catheter comprises a third set of at least two LEDs, wherein a first LED of said third set emits light at a different wavelength than a second LED of said third set and wherein a light receiver adapted to receive the reflected light of said third set generates a signal, which signal is processed to monitor cerebral cytochrome-c-oxidase and/or water content. Specifically, it is preferable that the first LED of said third set emits light with a wavelength of 520 nm and the second LED of said third set emits light with a wavelength of 550 nm for monitoring cerebral cytochrome-c-oxidase. Alternatively, the wavelength of the LEDs of the third set can be any of 740 nm, 840 nm and 960 nm for monitoring the water content.

According to the present invention, any of the first, second and third set can be provided without any of the other sets. However, any combination of two sets can be provided. Most preferably, the intracranial catheter comprises all three sets. Further, there can be provided one light receiver for each set or each LED or there can be provided one light receiver for all sets.

Further details and advantages of the present invention will be obtained from the following description of an embodiment and the accompanying drawings, in which:
- Figure 1: is a side view of an embodiment of the intracranial catheter,
- Figure 2: is an enlarged view of the tip element (detail X in figure 1), and
- Figure 3: is a partial cross-sectional view of the intracranial catheter being inserted into a craniospinal compartment and connected to a processing unit.

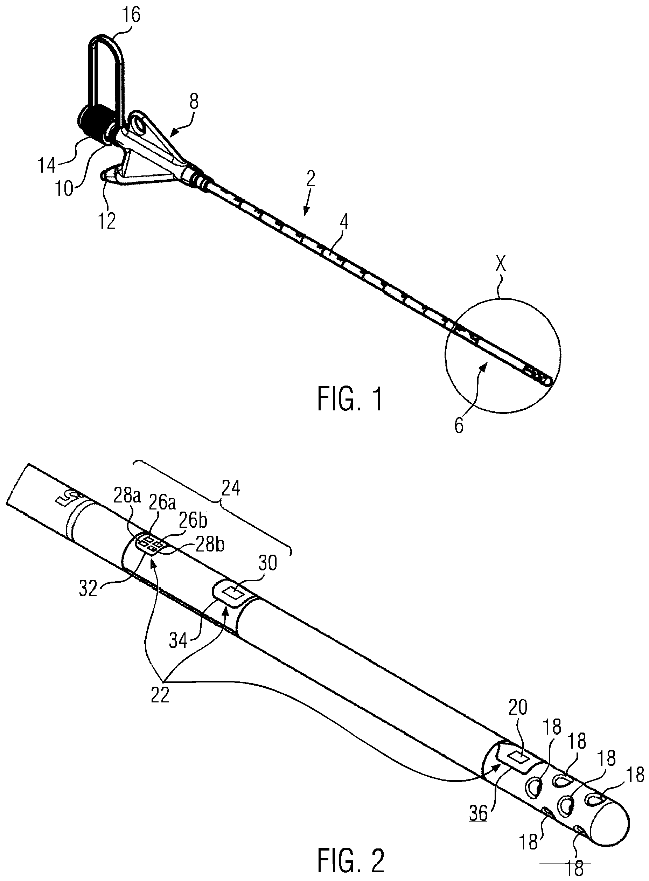

As shown in figure 1, the intracranial catheter 2 comprises a longitudinal body 4 that has a tip element 6 forming a distal end of said longitudinal body 4. A proximal end of the longitudinal body 4 is provided with a connector 8 comprising a fluid port 10 and a data port 12, wherein the fluid port 10 is connected to an opening of a drainage channel (not shown) formed in the longitudinal body 4 and the data port 12 is configured as an interface for transmitting sensor signals generated by the intracranial catheter 2 to a processing unit. The fluid port 10 is closed via a cap 14 that is secured to the connector 8 by a holding band 16. The drainage channel extends from the proximal end of said longitudinal body 4 to openings 18 of said tip element 6 (see figure 2). It permits drainage of the surrounding tissue through the intracranial catheter 2.

A pressure sensor device 20 of a sensor 22 is arranged near the openings 18 of the tip element 6. Further, the sensor 22 comprises optical sensor devices 24, which are: a first set 26 of two LEDs 26a, 26b, a second set 28 of two LEDs 28a, 28b, a third set of two LEDs (not shown) and a photodetector as a light receiver 30. Although the sensor 22 in this embodiment comprises a pressure sensor device and optical sensor devices, the sensor 22 may, in another embodiment, comprise only a pressure sensor device or only optical sensor devices and/or more than two LEDs per set and/or one, two or more than three sets of LEDs. In any case, the sensor 22 may additionally comprise a temperature sensor device. Further, more than one photodetector can be provided as a light receiver.

The pressure sensor device 20 and the optical sensor devices 24 are arranged in cavities that are formed in the tip element 6 and covered in a sealing manner by windows 32, 34, 36. Windows 32, 34 are translucent. Window 36 does not have to be translucent, if the pressure sensor device 20 is not an optical sensor device. The portion of the tip element 6 supporting the optical sensor devices 24 and the distal tip supporting the pressure sensor device 20 usually are stiff. The portion of the tip element 6 between the portion supporting the optical sensor devices 24 and the distal tip supporting the pressure sensor device 20 can be flexible, or inexistent.

Due to the absorption characteristic of indocyanine green and oxygenated and deoxygenated haemoglobin, it is preferable to use LEDs emitting light in the near infrared range. In this embodiment, the first LED 26a of the first set 26 emits light having a wavelength of 808 nm, the second LED 26b of the first set 26 emits light having a wavelength of 905 nm, the first LED 28a of the second set 28 emits light having a wavelength of 690 nm, the second LED 28b of the second set 28 emits light having a wavelength of 760 nm, the first LED of the third set emits light having a wavelength of 740 nm, and the second LED of the third set emits light having a wavelength of 840 nm.

Similarly as in noninvasive pulse oximetry, the cerebral oxygen saturation of a person is monitored by measuring the changing absorbance, reflectance and/or scattering of the two different wavelengths of at least one set of LEDs. Moreover, cerebral blood flow is monitored by measuring a pulsatile pressure curve using signals generated by the pressure sensor device 20 and/or optical signals and analysing said signals using pulse contour analysis employed by a processing unit. By combining the analysis results of the oxygen saturation measurement and the pressure curve measurement, monitoring of cerebral blood flow, preferably in terms of volume per time or in terms of volume per time per amount of cerebral tissue, for example in ml/min/100g cerebral tissue, is improved.

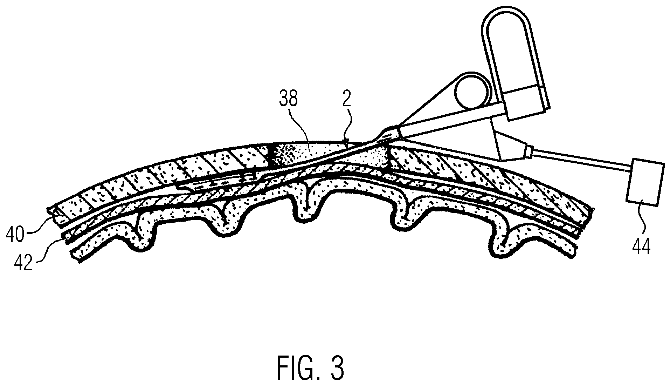

As shown in figure 3, the intracranial catheter 2 can be inserted through a burr hole 38 in the skull 40 and into a craniospinal compartment located between the dura mater 42 and the skull 40 (epidural), or under the dura mater 42 (subdural). An external processing unit 44 is connected to the data port 12 of the intracranial catheter 2. In another embodiment, the processing unit can be integrated into the intracranial catheter 2.

For the epidural or subdural application, the intracranial catheter 2 can be formed without a drainage channel. However, when the intracranial catheter 2 is inserted through a burr hole 38 in the skull 40 and all the way into a ventricle in the brain, i.e. using the intracranial catheter 2 as a ventricular probe, the intracranial catheter 2 preferably has a drainage channel.

### List of reference signs

- 2: intracranial catheter
- 4: longitudinal body
- 6: tip element
- 8: connector
- 10: fluid port
- 12: data port
- 14: cap
- 16: holding band
- 18: opening
- 20: pressure sensor device
- 22: sensor
- 24: optical sensor device
- 26a: first LED of the first set
- 26b: second LED of the first set
- 28a: first LED of the second set
- 28b: second LED of the second set
- 30: light receiver
- 32, 34: translucent window
- 36: window
- 38: burr hole
- 40: skull
- 42: dura mater
- 44: processing unit

## Claims

1. An intracranial catheter (2) for measuring a flow of blood through a body tissue, comprising a longitudinal body (4) adapted to be inserted into the craniospinal compartment, which longitudinal body (4) has a tip element (6), which forms a distal end of said longitudinal body (4), and preferably a channel extending from a proximal end of said longitudinal body (4) to at least one opening (18) of said tip element (6), **characterized by** a sensor (22) configured to detect a pulsatile pressure curve, which is analyzed via a pulse contour analysis performed by a processing unit (44) coupled to said sensor (22).

2. The intracranial catheter (2) according to claim 1,
wherein the sensor (22) comprises optical sensor devices (24) including at least one light emitter for emitting light, preferably in the near infrared range, into the body tissue and at least one light receiver (30) for receiving light reflected in the body tissue and back to the longitudinal body (4) and for generating a signal.

3. The intracranial catheter (2) according to claim 2, wherein the signal generated by the light receiver (30) comprises information about a difference between an oxyhemoglobin concentration and a deoxyhemoglobin concentration, which signal is processed to monitor continuous cerebral blood flow.

4. The intracranial catheter (2) according to any one of the preceding claims, **characterized by** a first set (26) of at least two LEDs (26a, 26b), wherein a first LED (26a) of said first set (26) emits light at a different wavelength than a second LED (26b) of said first set (26) and wherein said LEDs (26a, 26b) of said first set (26) are adapted to emit light in the near infrared range and wherein a light receiver (30) adapted to receive the reflected light of said first set (26) generates a signal, which signal is processed to monitor cerebral blood flow.

5. The intracranial catheter (2) according to any one of the preceding claims, **characterized by** a second set (28) of at least two LEDs (28a, 28b), wherein a first LED (28a) of said second set (28) emits light at a different wavelength than a second LED (28b) of said second set (28) and wherein said LEDs (28a, 28b) of said second set (28) are adapted to emit light in the near infrared range and wherein a light receiver (30) adapted to receive the reflected light of said second set (28) generates a signal, which signal is processed to monitor cerebral oxygen saturation.

6. The intracranial catheter (2) according to any one of the preceding claims, **characterized by** a third set of at least two LEDs, wherein a first LED of said third set emits light at a different wavelength than a second LED of said third set and wherein a light receiver (30) adapted to receive the reflected light of said third set generates a signal, which signal is processed to monitor cerebral cytochrome-c-oxidase and/or water content.
